# EUROPEAN PATENT SPECIFICATION

(11) **EP 1 059 294 B1**
(45) Date of publication and mention of the grant of the patent: **29.06.2005**
(21) Application number: 00500098.9
(22) Date of filing: 23.05.2000
(51) Int. Cl.: C07D 473/00, C02F 1/16, B01D 1/26

(54) **Procedure for the treatment of purines**
Verfahren zur Behandlung von Purinen
Procédé de traitement de purines

(30) Priority: 09.06.1999 ES 9901267
(43) Date of publication of application: 13.12.2000
(73) Proprietor: Guascor Investigacion y Desarrollo, S.A., 01510 Minano (Alava) (ES)
(72) Inventor: Arregui Ciarsolo, Juan Luis, 01192 Arkaute (Alava) (ES)
(74) Representative: Carpintero Lopez, Francisco

(56) References cited:
- WO-A-99/03785
- ES-A- 2 139 523
- CHEMICAL ABSTRACTS, vol. 122, no. 26, 26 June 1995 (1995-06-26) Columbus, Ohio, US; abstract no. 317379, CUADRA R.J.: "Process and installation for purine treatment" XP002147682 & ES 2 059 282 A (K.W.S.A.) 1 November 1994 (1994-11-01)

## Description

### OBJECT OF THE INVENTION

The invention relates to a procedure for the treatment of purines. In this procedure a separation of a liquid product and a slurry takes place, after which both products are duly heated and submitted to treatment phases to finally obtain a treated organic substance which can be used as fertiliser. The heat supplied for heating these products is obtained, in the commented patent of the main invention, from an energy plant with production of electrical energy and useful thermal energy (co-generation).

It is the object of this invention to introduce improvements in the phases of the procedure for treatment of purines, in particular a certain way of applying heat originating from the energy plant, with the particular characteristic that the heat which has to be supplied originates mainly from exhaust gases from the motors of the energy plant itself as well as from the heat liberated by the cooling radiators of these motors.

It is also object of this invention the use of digesters-dehydrators for the treatment of the liquid product in which both the dehydration and biological treatment of said liquid product takes place.

### BACKGROUND OF THE INVENTION

In the procedure corresponding to the Spanish patent of invention P9600968 some phases of treatment of purines are disclosed to avoid the strong smells normally generated by fermentation of purines to obtain fertilising organic residues, as well as to achieve a notable reduction in the treatment time required for the purines themselves.

Specifically, the procedure described in this main patent of invention is based on a forced drying of the purines, with heat being applied, avoiding the fermentation of the purine and, as a consequence, the generation of smells. Said forced drying process means that the time period required is reduced, and as a result of the process a treated product or organic product is obtained with no pathogenic elements or toxic salts which, therefore, maybe spread on the ground, either directly or enriched with mineral elements to provide a appropriate organic fertilisation.

The procedure itself includes a first phase in which the mechanical separation of the purine takes place to give a liquid product and, independently, a slurry. In a second phase the slurry is passed through an oven where it is heated to a temperature that is high enough to eliminate ammonia salts and other pathogenic elements, yielding a product that can be considered as a treated organic material at the output of the oven.

In another phase of the procedure, the liquid product is introduced into evaporators, where, also through the application of heat, a semi-dry residue is obtained which, after a mechanical separation, is added to the slurry obtained in the first phase and the drying of both components finished in an oven.

The heat supplied both to the oven and to the evaporators is obtained from an energy plant via thermal motors, using natural gas, propane, diesel, oil or any other combustible material which may be used in thermal machines as the combustible material.

Through this thermal procedure applied to the purines, a quick drying thereof is obtained, with no fermentations and smells. A notable reduction in the treatment time of the purines is also achieved.

In the application PCT ES98/00210, belonging to the same applicant, a device is disclosed for the treatment and elimination of aqueous liquids through controlled atmospheric action, according to which the liquid to be treated is distributed alternately over each face of contact blocks which allow the liquid to evaporate when hot air is passed through them, obtaining a dry residue which can be extracted using suitable means.

Patent ES-A-2 139 523 refers to an installation for processing and eliminating purines in a livestock farm which comprises means to separate solids from liquid, oxygenating means that place into contact the liquid with the air, spraying means for the purines evaporation and extracting means consisting of a fan that impels the sprayed liquid to the outside.

### DESCRIPTION OF THE INVENTION

The improvements applicable to the procedure referred to in the previous section are focussed mainly on the form of applying the heat from the energy plant, both to the slurry drying oven and to the liquid product or fraction evaporators, as well as on the fact that devices for liquid elimination described in application PCT ES98/00210 are used as evaporators. Hereinafter we will call these devices for liquid elimination digesters-dehydrators because, as we will see later, both the dehydration of the liquid and its biological treatment takes place in these devices and they also allows the treatment of the purines to be completed without the production of contaminating waste.

Other improvements relate to the incorporation of a cyclone at the output of the heating oven, which allows separation of the dry product from the gases and vapours, also using the heat from these gases and vapours for the dehydration of the liquid fraction in the digesters.

More specifically, the heat supplied to the drying oven of the slurry originates from the exhaust gases from the motors of the energy plant, with the particular characteristic that said motors will have their exhaust pipes connected to a single large channel which feeds the aforementioned drying oven with hot gases, leading to heating in two phases, namely in the first contact between the gases and the purine leading to spontaneous evaporation and immediately afterwards a slow and progressive drying, until 80% dry material is attained at the output of the oven.

Furthermore, heating of the liquid fraction in the digesters-dehydrators is carried out using the heat given off by the cooling radiators of the thermal motors of the energy plant which, for this reason, are placed within the digesters themselves.

Specifically, the cooling radiators of the motors corresponding to the energy plant are deployed on the side wall of the digesters such that the air flow sucked in by the central fan will pass first through these radiators and then, once warm, will act directly on the contact blocks, leading to dehydration of the liquid fraction.

Another improvement, as has been mentioned, relates to the fact that the evaporators consist, through digesters-dehydrators, of the type which are formed by contact panels on whose faces the liquid product to be evaporated is sprayed, carrying out the treatment such that the liquid fraction obtained in the mechanical separators is sent first to a regulating reservoir from where it is distributed, via a series of recycling pumps, to sprinklers which spray the liquid fraction over the contact blocks, thus carrying out the purine treatment with zero waste, in other words, no liquid waste is released outside the plant.

At the same time as the dehydration of the liquid fraction the biological treatment of the fraction also takes place, being carried out in the digesters themselves in spontaneous fashion, as this is favoured by the temperature in the digester and by the high degree of oxygenation of the liquid fraction duly spread out over the contact blocks. This treatment thus eliminates the need for the liquid fraction to pass through the typical biological treatment station with stirring and oxygen supply.

Thus, the product obtained in these digesters-dehydrators can be used as biological sludge for appropriate utilisation or introduced into the reception chute of the slurry, at the moment in which the separation of this product from the actual liquid corresponding to the purines in question takes place.

According to another of the improvements object of the invention, after the oven the incorporation of a cyclone is provided, into which the gas mixture, water vapour and dehydrated product are introduced, such that in this cyclone most of the solids are separated. These solids are extracted by means of a valve conveniently established in the bottom of the cyclone, constituting already a treated organic substance, which can be used as a ground fertiliser. They may be extracted directly from the plant in granular form or may pass through a pelleting machine to obtain the corresponding pellets. This corrected organic substance may be enriched with organo-mineral substances or other additives with a view to obtain fertilisers with certain characteristics.

When necessary, part of this corrected organic substance obtained is mixed with the slurry to reduce the humidity of the mixture that enters the drying oven and so prevent the slurry from sticking to the walls of the oven.

The remaining particles in suspension and the mixture of gasses and water vapour are sent, via a centrifugal fan, to the digesters where the gasses are scrubbed and whose heat, in turn, is used to heat part of the liquid product on the contact blocks.

For these purposes, no radiator has been provided in the external sector or zone of the digester. Instead, there is an input for the hot gases originating from the cyclone, such that these gases, on being swept along by the central fan, act directly on the contact block corresponding to this sector or zone of the digester.

Thus, one of the contact blocks of each digester is heated by the hot gases from the cyclone, while the rest of the contact blocks are heated by hot air from the radiators of the motors of the energy plant. Obviously, if there is no cyclone then the heating will be effected by the radiators of the motors only.

### DESCRIPTION OF THE FIGURES

To complete the description being made here and with the aim of providing a better understanding of the characteristics of the invention, the present description is accompanied, as an integral part thereof, by a set of drawings in which, by way of illustration and in non-limiting fashion, the following has been represented:

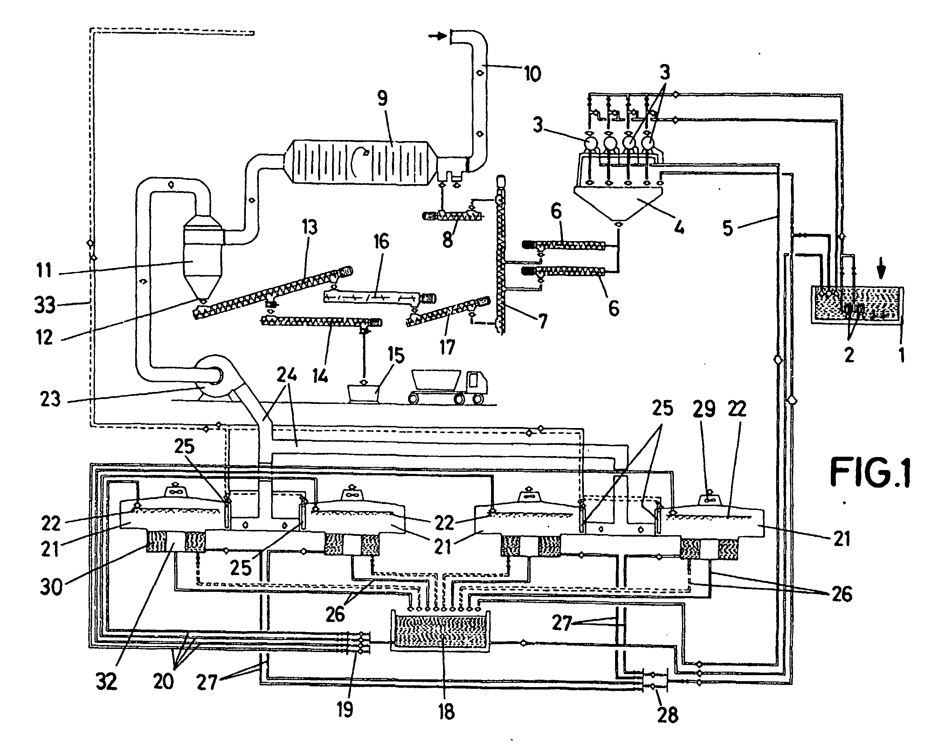

Figure 1: Shows a schematic representation corresponding to the installation for putting into practice the procedure for the treatment of purines with the improvements object of the invention.
Figure 2: Shows a partially sectioned side projection of a pair of digesters-dehydrators of the type that are present in the installation represented in the previous figure.

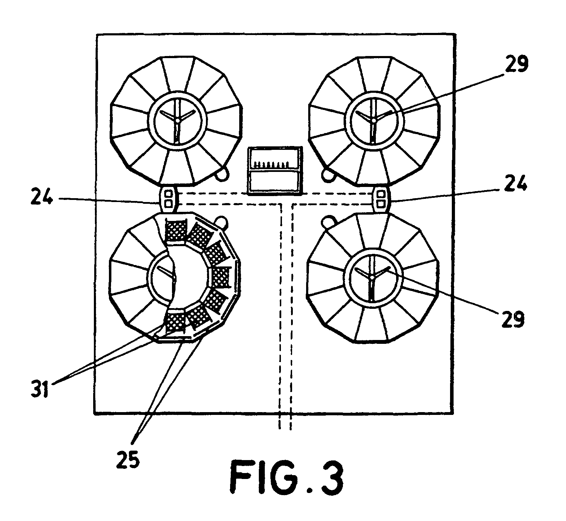

Figure 3: Shows a plan view of an installation consisting of four digesters-dehydrators, each one including a series of radiators of the cooling circuit for the motors as well as the input for hot gases originating from the circuit.

### PREFERRED EMBODIMENT OF THE INVENTION

In view of these figures it can be seen how, in accordance with the proposed procedure, the purines arrive in a reception reservoir (1) from where, via appropriate pumps (2), they are fed to some mechanical separators (3), which separate the slurry from the liquid product, such that the slurry is transferred directly to a chute (4), while the liquid product leaves via a channel (5). This slurry arrives, from the chute (4), at some transporters (6) and then at another transporter (7) where, optionally, it can be mixed with part of the treated organic product obtained as a result of the treatment. This mixture reaches a feeder (8) from where it passes into a thermal drying oven (9), such that the heating of the oven to dry the slurry is carried out using the exhaust gases originating from the motors of an energy plant, such that all these motors are connected to a common collector (10) via which the circulation of gases from all of them towards the thermal drying oven takes place (9).

Thus a dehydrate of the slurry is obtained in this oven (9), such that the mixture of gases, water vapour and dehydrated product is made to pass to a cyclone (11) located after the oven (9), and in which most of the solid particles in suspension contained in the gas are eliminated, the extraction of the solid product, consisting of a treated organic substance, being effected through a valve located at the bottom (12) of this cyclone (11). The product enters some transporters (13) from where, and via intermediate transport elements (14), the product can enter an external container, or else enter a pelleting machine in order to obtain the corresponding pellets.

Optionally, part of the treated organic substance obtained can pass through a removing device (16) and from there to a transporter (17-7) with a view to mixing this dry residue with the initial slurry from the chute (4), thus preventing the slurry from sticking to the walls of the oven.

Furthermore, the liquid product obtained in the mechanical separators (3), and via the channel (5), reaches a regulating reservoir (18) from where it is distributed, using appropriate distribution means (19), through the channels (20), to respective digesters-dehydrators (21), in which the dehydration of the liquid product and biological treatment takes place, producing the evaporation on contact blocks (known in their own right) which are sprayed by some sprinklers (22) which spray this liquid product originating from the regulating reservoir (18).

To improve the evaporation conditions on the contact blocks, and consequently to increase the capacity for dehydration of the liquid product in the digesters (21), it is provided that radiators (25) of the thermal motors of the energy plant are deployed along the edge.

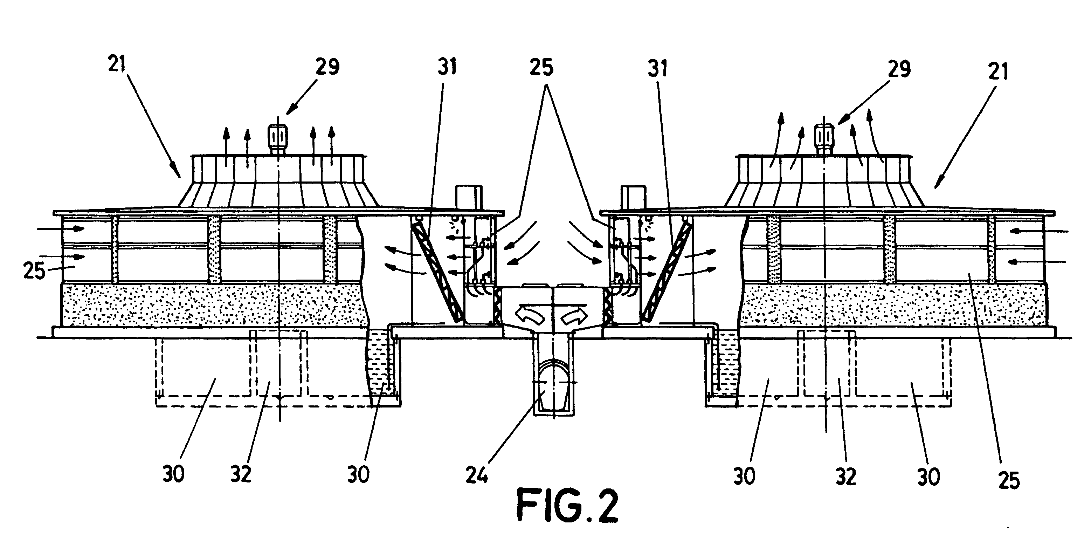

Specifically, figure 2 shows a side elevation of the digesters (21) on whose lateral wall the radiators (25) are deployed, such that the air sucked in by the central fan (29) passes through the radiators, being heated in the process, to then act on the contact blocks (31) established inside the digesters themselves (21), according to a tilted arrangement as shown clearly in figure 2.

This arrangement of radiators of the thermal motors also has the advantage that the water that circulates inside the radiators (25) is cooled and thus the corresponding cooling of the thermal motors belonging to the energy plant is achieved.

It has also been provided that no radiator (25) is incorporated into an external sector or zone of the digester (21), so that instead there is a hot gas input (24), originating from the cyclone, such that these gases, on being swept along by the central fan (29), act directly on the contact block (31) corresponding to this sector or zone of the digester (21).

In this way, the contact block (31) facing the gas input (24) is heated by the hot gases originating from the cyclone (11), while the remaining contact blocks (31) are heated by hot air from the radiators (25) of the motors of the energy plant.

As a result of the dehydration that occurs in the contact blocks (31) biological sludges are obtained which are deposited at the bottom of the digesters (21). These digesters have their bases divided into two zones, one of which constitutes an annular peripheral reservoir (30), in the most external zone, and another central, constituting a circular reservoir (32), such that the sludges fall in the peripheral reservoir (30), with the liquid part thereof overflowing into the central reservoir (32) from where they are recycled to the regulating reservoir (18), while the solid part of the biological sludges are extracted from the peripheral reservoir (30), via the channels (27), to some purging means (28), from where they are transferred to the chute (4) where they form part of the slurry obtained in the first phase of separation of the process.

In this fashion, the treatment of the purines is carried out with zero waste, in other words, there are no liquid wastes released to the environment, wastes which are obviously not desirable as these liquids contain contaminating particles.

The circuit of water circulation corresponding to the cooling radiators (25), is that referred to by (33), in which the input direction has been indicated with an arrow, as has the input direction of the gases in the common collector (10) corresponding to the exhaust gases of the motors of the energy plant itself.

## Claims

1. Procedure for the treatment of purines, in which the purines arriving from the reception reservoir (1) are submitted to a separation phase by mechanical means (3) to obtain a slurry and a liquid product, the slurry being transferred to a thermal drying oven (9), while the liquid product is sent to some evaporators (21), with the heat supplied both to the drying oven (9) and the evaporators (21) being obtained from an energy plant with thermal motors operating on any combustible material, **characterised in that** the heating of the slurry, in the thermal drying oven (9), is produced by exhaust gases of the motors of the energy plant, through a common collector (10) connected to said drying oven (9), while the dehydration of the liquid product is effected in digesters-dehydrators (21), by heat supplied by the radiators (25) corresponding to the water cooling circuit of the thermal motors themselves of the energy plant.

2. Procedure for the treatment of purines according to claim 1, **characterised in that** the digesters-dehydrators (21) incorporate contact blocks (31) sprayed using sprinklers (22), such that the liquid fraction obtained in the mechanical separators (3) is dispatched, via a channel (5) to a regulating reservoir (18) from where it is distributed to the sprinklers (22) which spray the liquid fraction over the contact blocks (31), obtaining a residue, whose liquid part is recycled once more to the regulating reservoir (18) while the solid part is sent to the chute (4) to so form part of the slurry obtained in the mechanical separators (3), thus completely preventing the release of liquid waste to the environment.

3. Procedure for the treatment of purines, according to the previous claims, **characterised in that** the radiators (25) are deployed over the side surface and concentrically on the wall of the digesters (21), such that the air sucked in by the central fan (29) will pass through these radiators (25), undergoing a heating, and cooling the water circulating through the radiators (25) to thus achieve cooling of the motors of the energy plant, with the air heated by the radiators passing through the contact blocks (31) leading to dehydration of the liquid fraction.

4. Procedure for the treatment of purines, according to previous claims, **characterised in that** the digesters (21) have a base divided into two zones, one which constitutes an annular reservoir (30), in the outermost part, and another central which constitutes a circular reservoir (32), such that the residue resulting from dehydration of the liquid fraction on the contact blocks, falls into the annular reservoir (30), the liquid part thereof spilling over into the central reservoir (32), from where it is recycled to the regulating reservoir (18), while the solid part or biological sludges are extracted from the peripheral reservoir (30), via some channels (27), to some purging means (28) which send them back to the chute (4).

5. Procedure for the treatment of purines, according to previous claims **characterised in that** biological treatment of the liquid fraction takes place in the digesters-dehydrators (21), obtaining a biological sludge as a dry residue, this treatment being effected spontaneously on the contact blocks due to the temperature of the digester and to the large degree of oxygenation of the liquid fraction as a result of being spread out on the contact blocks.

6. Procedure for the treatment of purines, according to previous claims **characterised in that** the mixture of gases, water vapour and product dehydrated in the oven (9) is passed through a cyclone (11) where a large part of the solids in suspension contained in the gas are eliminated and the solid part of the product is extracted for subsequent transport and definitive use, while the mixture of gases and water vapour passes, via a centrifugal fan (23), to a channel (24), from where it arrives at the digesters (21) producing simultaneous gas scrubbing and heating and dehydration of the liquid fraction applied to the contact blocks (31) which are facing the aforementioned gas input, with the possibility of removing the corresponding radiator (25) from this zone of the digester.

## Patentansprüche

1. Verfahren zur Behandlung von Purinen, bei dem die Purine, die aus dem Aufnahmebecken (1) ankommen, einer Trennphase mit mechanischen Mitteln (3) unterworfen werden, um eine Aufschlämmung und ein flüssiges Produkt zu erhalten, wobei die Aufschlämmung zu einem thermischen Trocknungsofen (9) überführt wird, während das flüssige Produkt zu einigen Verdampfern (21) geleitet wird, wobei die Wärme, die sowohl dem Trocknungsofen (9) als auch den Verdampfern (21) zugeführt wird, aus einem Kraftwerk mit Wärmemotoren, die mit irgendeinem brennbaren Material arbeiten, erhalten wird, **dadurch gekennzeichnet, daß** das Erhitzen der Aufschlämmung, im thermischen Trocknungsofen (9), durchgeführt wird durch Abgase der Motoren des Kraftwerkes, die durch eine gemeinsame Sammelleitung (10) mit besagtem Trocknungsofen (9) verbunden sind, während die Entwässerung des flüssigen Produktes in Faulbehältern-Entwässerern (21) bewirkt wird, durch Hitze, die von den Radiatoren (25) zugeführt wird, die dem Wasserkühlkreislauf der Wärmemotoren selbst des Kraftwerkes entsprechen.

2. Verfahren zur Behandlung von Purinen nach Anspruch 1, **dadurch gekennzeichnet, daß** die Faulbehälter-Entwässerer (21) Kontaktblöcke (31) umfassen, die unter Verwendung von Sprinklern (22) besprüht werden, so daß die flüssige Fraktion, die in den mechanischen Trennern (3) erhalten wird, über einen Kanal (5) zu einem Regulierungsbecken (18) abgegeben wird, aus dem sie zu den Sprinklern (22) verteilt wird, die die flüssige Fraktion über die Kontaktblöcke (31) sprühen, wodurch ein Rückstand erhalten wird, dessen flüssiger Teil noch einmal zum Regulierungsbecken (18) rückgeführt wird, während der feste Teil zur Schütte (4) transportiert wird, um so Teil der Aufschlämmung zu bilden, die in den mechanischen Trennern (3) erhalten wird, wodurch die Freisetzung von Flüssigabfall in die Umwelt vollständig verhindert wird.

3. Verfahren zur Behandlung von Purinen nach den vorangehenden Ansprüchen, **dadurch gekennzeichnet, daß** die Radiatoren (25) über die Seitenfläche und konzentrisch auf der Wand der Faulbehälter (21) verteilt sind, so daß die durch den zentralen Ventilator (29) eingesaugte Luft durch diese Radiatoren (25) strömen wird, wobei sie eine Erhitzung durchläuft, und das Wasser, das durch die Radiatoren (25) zirkuliert, abgekühlt wird, um so eine Abkühlung der Motoren des Kraftwerkes zu erreichen, wobei die von den Radiatoren erhitzte Luft durch die Kontaktblöcke (31) strömt, was zur Entwässerung der flüssigen Fraktion führt.

4. Verfahren zur Behandlung von Purinen nach den vorangehenden Ansprüchen, **dadurch gekennzeichnet, daß** die Faulbehälter (21) eine Basis besitzen, die in zwei Zonen aufgeteilt ist, von denen eine ein Ringbecken (30) darstellt, im äußeren Teil, und eine weitere zentrale, die ein Kreisbecken (32) darstellt, so daß der Rückstand der aus der Entwässerung der flüssigen Fraktion auf den Kontaktblöcken resultiert, in das Ringbecken (30) fällt, wobei der flüssige Teil davon ins Zentralbecken (32) überläuft, von dem er in das Regulierungsbecken (18) rückgeführt wird, während der feste Teil oder die biologischen Schlämme aus dem Randbecken (30) über einige Kanäle (27) zu einigen Spüleinheiten (28) herausgezogen werden, die sie zurück zur Schütte (4) leiten.

5. Verfahren zur Behandlung von Purinen nach den vorangehenden Ansprüchen, **dadurch gekennzeichnet, daß** die biologische Behandlung der flüssigen Fraktion in den Faulbecken-Entwässerern (21) stattfindet, wodurch ein biologischer Schlamm als ein trockener Rückstand erhalten wird, wobei diese Behandlung spontan auf den Kontaktblöcken aufgrund der Temperatur des Faulbecken und des hohen Grades an Sauerstoffbeladung der flüssigen Fraktion, als ein Ergebnis davon, daß sie auf den Kontaktblöcken verteilt wird, bewirkt wird.

6. Verfahren zur Behandlung von Purinen nach den vorangehenden Ansprüchen, **dadurch gekennzeichnet, daß** die Mischung aus Gasen, Wasserdampf und im Ofen (9) entwässertem Produkt durch einen Zyklon (11) geleitet wird, in dem ein großer Teil der Feststoffe in Suspension, die im Gas enthalten sind, eliminiert wird und der feste Teil des Produktes für anschließenden Transport und Endverwendung herausgezogen wird, während die Mischung aus Gasen und Wasserdampf über einen Zentrifugalventilator (23) zu einem Kanal (24) geleitet wird, aus dem er an den Faulbehältern (21) ankommt, wodurch gleichzeitige Gaswäsche und Erhitzen und Entwässerung der flüssigen Fraktion, die auf die Kontaktblöcke (31) aufgebracht wird, die vorgenanntem Gaseintrag zugewandt ist, erzeugt wird, mit der Möglichkeit der Entfernung des entsprechenden Radiators (25) aus dieser Zone des Faulbehälters.

## Revendications

1. Procédure pour le traitement de purines, dans laquelle les purines provenant du réservoir de réception (1) sont soumises à une phase de séparation par des moyens mécaniques (3) afin d'obtenir une pâte et un produit liquide, la pâte étant transférée à un four de séchage thermique (9), tandis que le produit liquide est envoyé à des évaporateurs (21), la chaleur délivrée à la fois au four de séchage (9) et aux évaporateurs (21) étant obtenue à partir d'une installation énergétique comportant des moteurs thermiques fonctionnant avec n'importe quelle matière combustible, **caractérisée en ce que** le chauffage de la pâte, dans le four de séchage thermique (9), est produit par des gaz d'échappement des moteurs de l'installation énergétique, par l'intermédiaire d'un collecteur (10) commun relié audit four de séchage (9), tandis que la déshydratation du produit liquide est effectuée dans des digesteurs-déshydrateurs (21), par la chaleur délivrée par les radiateurs (25) correspondant au circuit de refroidissement par eau des moteurs thermiques eux-mêmes de l'installation énergétique.

2. Procédure pour le traitement de purines selon la revendication 1, **caractérisée en ce que** les digesteurs-déshydrateurs (21) incorporent des blocs de contact (31) aspergés en utilisant des appareils d'aspersion (22), de sorte que la fraction liquide obtenue dans les séparateurs mécaniques (3) soit envoyée, par l'intermédiaire d'un canal (5), vers un réservoir de régulation (18) à partir duquel elle est répartie entre les appareils d'aspersion (22) qui pulvérisent la fraction liquide sur les blocs de contact (31), obtenant un résidu, dont la partie liquide est recyclée une fois de plus vers le réservoir de régulation (18) tandis que la partie solide est envoyée vers la goulotte (4) pour faire ainsi partie de la pâte obtenue dans les séparateurs mécaniques (3), évitant ainsi complètement la libération de déchets liquides dans l'environnement.

3. Procédure pour le traitement de purines, selon les revendications précédentes, **caractérisée en ce que** les radiateurs (25) sont déployés sur la surface latérale et concentriquement sur la paroi des digesteurs (21), de sorte que l'air aspiré par le ventilateur central (29) passe à travers ces radiateurs (25), subissant un chauffage, et refroidissant l'eau circulant dans les radiateurs (25) pour ainsi obtenir un refroidissement des moteurs de l'installation énergétique, l'air chauffé par les radiateurs qui passe à travers les blocs de contact (31) conduisant à une déshydratation de la fraction liquide.

4. Procédure pour le traitement de purines, selon les revendications précédentes, **caractérisée en ce que** les digesteurs (21) comportent une base divisée en deux zones, une qui constitue un réservoir annulaire (30), dans la partie la plus à l'extérieure, et une autre centrale qui constitue un réservoir circulaire (32), de sorte que le résidu résultant d'une déshydratation de la fraction liquide sur les blocs de contact tombe dans le réservoir annulaire (30), la partie liquide de celle-ci se déversant dans le réservoir central (32), d'où elle est recyclée vers le réservoir de régulation (18), tandis que la partie solide ou les boues activées sont extraites du réservoir périphérique (30), par l'intermédiaire de canaux (27), vers des moyens de purge (28) qui les renvoient vers la goulotte (4).

5. Procédure pour le traitement de purines, selon les revendications précédentes, **caractérisée en ce qu'**un traitement biologique de la fraction liquide a lieu dans les digesteurs-déshydrateurs (21), obtenant une boue activée en tant que résidu sec, ce traitement étant effectué spontanément sur les blocs de contact du fait de la température du digesteur et du degré élevé d'oxygénation de la fraction liquide en conséquence de sa dispersion sur les blocs de contact.

6. Procédure pour le traitement de purines, selon les revendications précédentes, **caractérisée en ce que** le mélange de gaz, de vapeur d'eau et de produit déshydraté dans le four (9) est passé à travers un cyclone (11) où une grande part des matières solides en suspension contenues dans le gaz sont éliminées et la partie solide du produit est extraite pour un transport subséquent et une utilisation définitive, tandis que le mélange de gaz et de vapeur d'eau passe, par l'intermédiaire d'un ventilateur centrifuge (23), vers un canal (24), d'où il arrive dans les digesteurs (21) produisant simultanément un lavage des gaz et un chauffage et une déshydratation de la fraction liquide appliquée sur les blocs de contact (31) qui sont face à l'entrée de gaz susmentionnée, avec la possibilité de retirer le radiateur (25) correspondant de cette zone du digesteur.
